# EUROPEAN PATENT APPLICATION

(11) **EP 4 407 028 A1**
(43) Date of publication of application: **31.07.2024**
(21) Application number: 21958400.0
(22) Date of filing: 24.09.2021
(51) Int. Cl.: C12N 15/09

(54) **METHOD FOR PRODUCING MODIFIED HUMAN INDUCED PLURIPOTENT STEM CELLS**

(71) Applicant: Cira Foundation, Kyoto-shi, Kyoto 606-8397 (JP)
(72) Inventor: TSUKAHARA, Masayoshi, Kyoto-shi, Kyoto 606-8397 (JP); YAMAMOTO, Yuko, Kyoto-shi, Kyoto 606-8397 (JP); KATO, Tomoaki, Kyoto-shi, Kyoto 606-8397 (JP); NAKASHIMA, Yoshiki, Kyoto-shi, Kyoto 606-8397 (JP)
(74) Representative: Cabinet Becker et Associés
(86) International application number: PCT/JP2021/035047
(87) International publication number: WO 2023/047524

(57) **Abstract**

A method for producing modified human induced pluripotent stem cells (iPSCs) in which two or more target genes have been modified, the production method including:
an introduction step of introducing CRISPR/Cas9 protein and a plurality of kinds of gRNAs for the two or more target genes into human iPSCs.

## Description

### [Technical Field]

The present invention relates to a method for producing modified human induced pluripotent stem cells (iPSCs). More specifically, the present invention relates to a method for producing modified human iPSCs in which two or more target genes have been modified, modified human iPSCs, differentiated cells induced from the modified human iPSCs, and a kit for modifying two or more target genes in human iPSCs.

### [Background Art]

In a case of allogeneic transplantation in which a donor cell is transplanted into a recipient (patient) who is another person, the transplanted cell is rejected due to an immune reaction.

The protein that plays the most important role in distinguishing between self-cells and non-self-cells is a major histocompatibility complex (MHC) or a cell surface protein called a human leukocyte antigen (HLA).

HLAs are classified into Class I and Class II. Class I HLA protein is expressed in most cell types in a body. The Class 1 HLA protein has a function of forming a heterodimer with a β2-microglobulin (B2M) to be expressed on a cell surface, and presenting a peptide with respect to a CD8-positive cytotoxic T cell to induce activation. The antigenic peptide presented is endogenous and has a length of 8 to 10 amino acids in many cases.

The main six genes classified into Class I HLA are an HLA-A gene, an HLA-B gene, an HLA-C gene, an HLA-E gene, an HLA-F gene, and an HLA-G gene. In addition, many pseudogenes (HLA-H, HLA-J, HLA-K, HLA-L, HLA-P, HLA-T, HLA-U, HLA-V, HLA-W, HLA-X, HLA-Y, and the like) are also known. Among the genes, three genes, namely the HLA-A gene, the HLA-B gene, and the HLA-C gene, have particularly high sequence diversity among individuals and play a major role in identifying self-cells and non-self-cells in transplantation immunity.

Class II HLA protein is mainly expressed in immune cells such as macrophages, dendritic cells, activated T cells, and B cells. The Class II HLA protein has a function of forming a heterodimer with an α chain and a β chain, and presenting a peptide with respect to a CD4 helper T cell to induce activation. The antigenic peptide presented is exogenous and has a length of 15 to 24 amino acids in many cases.

Genes classified into Class II HLA are HLA-DR (α chain: HLA-DRA, β chain: HLA-DRB), HLA-DQ (α chain: HLA-DQA1, β chain: HLA-DQB1), and HLA-DP (α chain: HLA-DPA1 or HLA-DPA2, β chain: HLA-DPB1 or HLA-DPB2). It is also known that many other pseudogenes (HLA-DMA, HLA-DMB, HLA-DOA, and HLA-DOB) are present.

The HLA genes have sequence diversity, and are thus involved in recognition of self-cells and non-self-cells at the cellular level. HLA matching is also particularly important for reducing immune rejection during allogeneic transplantation. For example, in transplantation of hematopoietic stem cells, it is recommended to find and transplant from a donor in which antigenicities in both alleles of HLA-A, HLA-B, HLA-C, and HLA-DR, that is, alleles at a total of 8 loci, are as consistent as possible.

Moreover, in consideration of the results of engraftment rates of kidney transplantation and the like, it has been reported that, as a degree of consistency of HLA antigenicity becomes high, the engraftment efficiency becomes significantly high.

Meanwhile since pluripotent stem cells such as human induced pluripotent stem cells (iPSCs) and embryonic stem cells (ESCs) have pluripotency capable of being differentiated into various cell types, cell therapy and regenerative medicine in which the pluripotent stem cells are differentiated into various cells and then transplanted to patients are attracting attention.

Actually, there have been reported cases where ESCs are differentiated into nerve cells and transplanted into a patient with a bone marrow injury, and cases where iPSCs are differentiated into retinal pigment epithelial cells and transplanted into a patient with age-related macular degeneration. It has been reported that in a case of transplanting such pluripotent stem cell-derived cells, it is important to match the transplanted cells with the HLA type of a patient.

However, pluripotent stem cells require a great deal of cost and time for their establishment. Therefore, there is a problem in that it is difficult to prepare cells matching the HLA type for each patient. As one of means for solving this problem, for example, Patent Document 1 discloses cells in which a B2M gene required for presenting Class I HLA protein on the cell surface has been deleted.

### [Citation List]

### [Patent Documents]

[Patent Document 1]
   PCT International Publication No. WO2012/145384
[Patent Document 2]
   PCT International Publication No. WO2019/160077

### [Non-Patent Document]

[Non-Patent Document 1]
Huaigeng Xu, et. al, Targeted Disruption of HLA Genes via CRISPR-Cas9 Generates iPSCs with Enhanced Immune Compatibility, Cell Stem Cell 24, 566-578, 2019.

### [Summary of Invention]

### [Technical Problem]

Since a B2M gene has been deleted in the cells disclosed in Patent Document 1, Class 1 HLA protein is not presented on the cell surface. Accordingly, it is considered that an immune reaction in a case where the cells are allogeneically transplanted is inhibited. However, in a case where the HLA protein is not presented on the cell surface, an antigen-presenting ability of the cells is lost. In a case where the cells lose their antigen-presenting ability, they cannot present virus- or tumor-derived antigens, for example, upon infection with viruses, or upon development into tumors. As a result, there is a risk in which the cells may help growth of the viruses or the tumors. In addition, cells in which HLA protein is not presented on the cell surface are attacked by NK cells that recognize "missing self."

Therefore, it is considered to selectively disrupt only optional HLA genes using a genome editing technique. However, a plurality of the HLA genes are present and many pseudogenes are included, and thus, there is a high degree of sequence homology between the HLA genes. In a case where a plurality of target genes are present as described above, if genome editing of each gene is performed individually, time and effort taken for producing desired cells are increased.

An object of the present invention is to provide a technique for producing modified human iPSCs in which two or more target genes have been modified.

### [Solution to Problem]

The present invention includes the following aspects.
[1] A method for producing modified induced pluripotent stem cells (iPSCs) in which two or more target genes have been modified, the production method including:
   an introduction step of introducing CRISPR/Cas9 protein and a plurality of kinds of gRNAs for the two or more target genes into human iPSCs.
[2] The production method according to [1],
   in which the human iPSCs are blood cell-derived iPSCs.
[3] The production method according to [1] or [2],
   in which in the human iPSCs, HLA-A genes or HLA-B genes are homozygous.
[4] The production method according to [3],
   in which in the human iPSCs, haplotypes of the HLA-A genes include A24:02 and haplotypes of the HLA-B genes include B52:01.
[5] The production method according to [3] or [4],
   in which in the human iPSCs, HLA-C genes are homozygous.
[6] The production method according to any one of [3] to [5],
   in which in the human iPSCs, HLA-DRB1 genes are homozygous.
[7] The production method according to any one of [1] to [6],
   in which the introduction step is performed by an electroporation method.
[8] The production method according to any one of [1] to [7],
   in which the two or more target genes are genes selected from the group consisting of HLA-A genes, HLA-B genes, HLA-C genes, HLA-DR genes, and Class II major histocompatibility complex transactivator (CIITA) genes.
[9] The production method according to any one of [1] to [8],
   in which the gRNAs are a gRNA consisting of a base sequence set forth in SEQ ID NO: 1 or a base sequence in which one or several bases are deleted, substituted, or added at a 5' end of the base sequence set forth in SEQ ID NO: 1, and a gRNA consisting of a base sequence set forth in SEQ ID NO: 2 or a base sequence in which one or several bases are deleted, substituted, or added at a 5' end of the base sequence set forth in SEQ ID NO: 2.
[10] The production method according to any one of [1] to [9],
   in which in the introduction step, the CRISPR/Cas9 protein and the gRNAs are introduced in a form of a complex.
[11] The production method according to any one of [1] to [10],
   in which in the introduction step, the CRISPR/Cas9 protein and the plurality of kinds of gRNAs are introduced at a mass ratio of a mass of the CRISPR/Cas9 protein:a total mass of the plurality of kinds of the gRNAs = 5:1 to 1:10.
[12] The production method according to [11],
   in which in the introduction step, the CRISPR/Cas9 protein is introduced in an amount of 1 to 50 µg per 1 × 10⁵ to 3 × 10⁶ cells of the human iPSCs.
[13] The production method according to any one of [1] to [12],
   in which the modification of the target genes is disruption of the target genes.
[14] Modified human iPSCs or differentiated cells induced from the modified human iPSCs, the cells including:
   disrupted HLA-A genes;
   disrupted HLA-B genes; and
   disrupted CIITA genes,
   in which the disrupted HLA-A genes or the disrupted HLA-B genes are homozygous,
   haplotypes of the disrupted HLA-A genes include A24:02, and
   haplotypes of the disrupted HLA-B genes include B52:01.
[15] Modified human iPSCs or differentiated cells induced from the modified human iPSCs, the cells including:
   disrupted HLA-A genes;
   disrupted HLA-B genes; and
   disrupted C11TA genes,
   in which haplotypes of HLA-C genes include C12:02.
[16] A kit for modifying two or more target genes in human iPSCs by introducing into the human iPSCs, the kit including:
   CRISPR/Cas9 protein; and
   a plurality of kinds of gRNAs for the two or more target genes.
[17] The kit according to [16],
   in which the introduction is performed by an electroporation method.
[18] The kit according to [16] or [17],
   in which the two or more target genes are genes selected from the group consisting of HLA-A genes, HLA-B genes, HLA-C genes, HLA-DR genes, and CIITA genes.
[19] The kit according to any one of [16] to [18],
   in which the gRNAs are a gRNA consisting of a base sequence set forth in SEQ ID NO: 1 or a base sequence in which one or several bases are deleted, substituted, or added at a 5' end of the base sequence set forth in SEQ ID NO: 1, and a gRNA consisting of a base sequence set forth in SEQ ID NO: 2 or a base sequence in which one or several bases are deleted, substituted, or added at a 5' end of the base sequence set forth in SEQ ID NO: 2.
[20] The kit according to any one of [16] to [19],
   in which the kit is used to introduce the CRISPR/Cas9 protein and the plurality of kinds of the gRNAs into the human iPSCs at a mass ratio of a mass of the CRISPR/Cas9 protein:a total mass of the plurality of kinds of the gRNAs = 5:1 to 1:10.
[21] The kit according to [20],
   in which the kit is used to introduce the CRISPR/Cas9 protein in an amount of 1 to 50 µg per 1 × 10⁵ to 3 × 10⁶ cells of the human iPSCs.

### [Advantageous Effects of Invention]

According to the present invention, it is possible to provide a technique for producing modified human iPSCs in which two or more target genes have been modified.

### [Brief Description of Drawings]

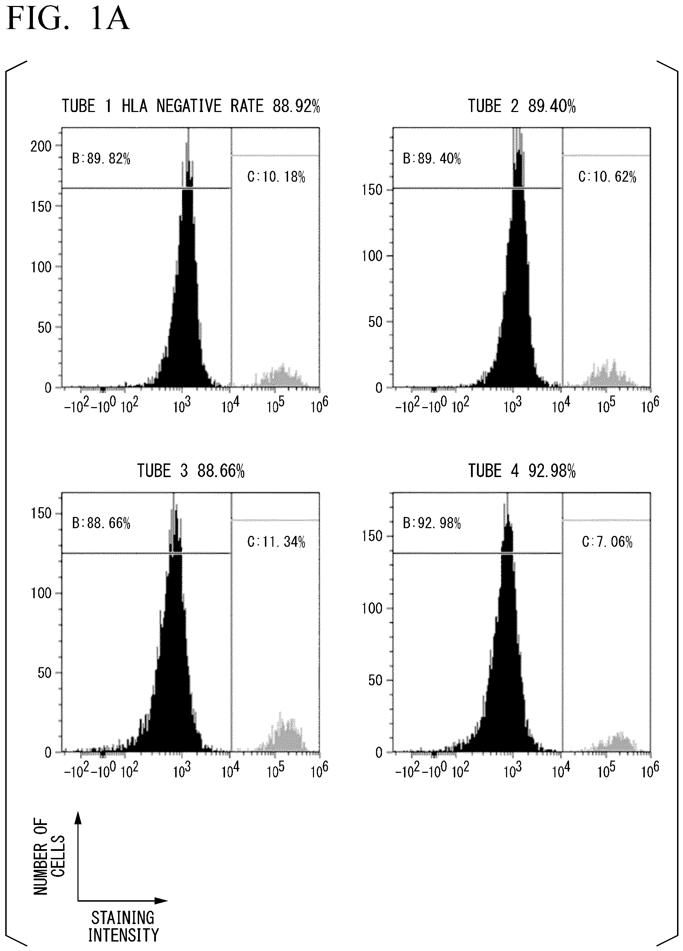

FIG. 1A is a graph showing the results of flow cytometric analysis in Examples.

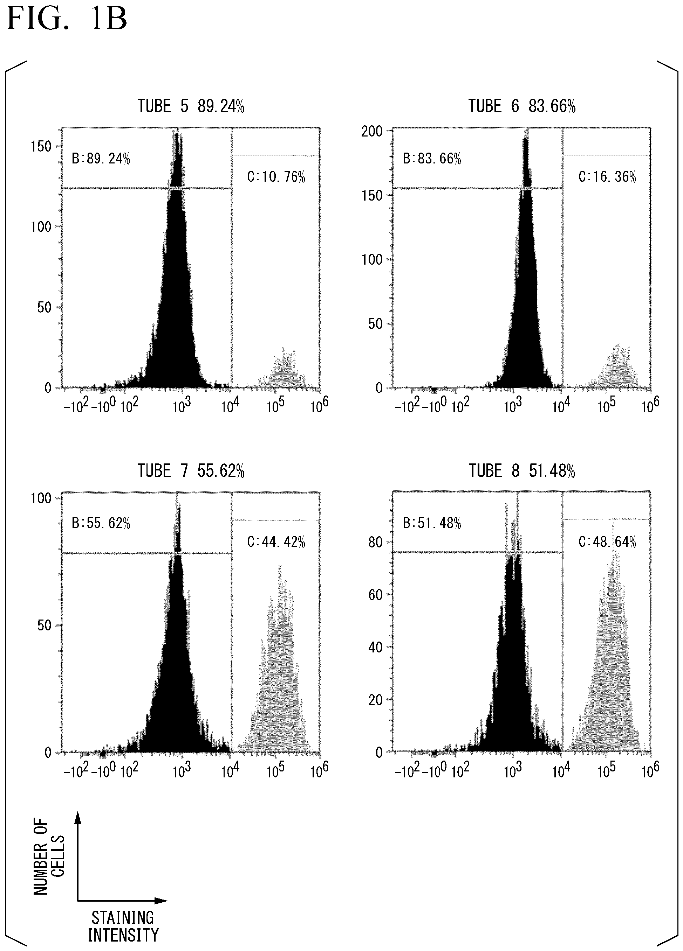

FIG. 1B is a graph showing the results of flow cytometric analysis in Examples.

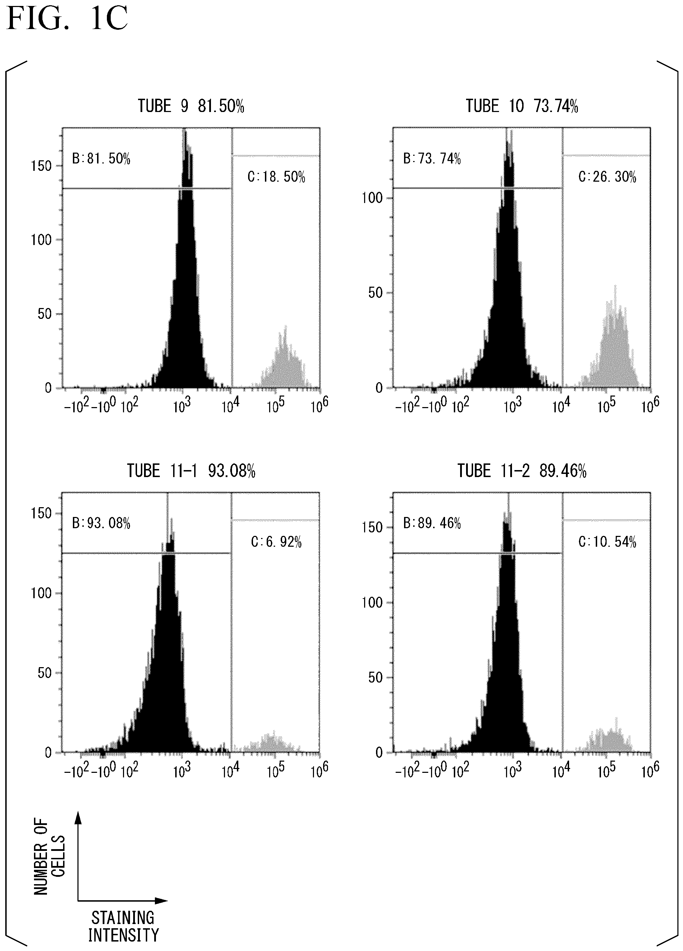

FIG. 1C is a graph showing the results of flow cytometric analysis in Examples.

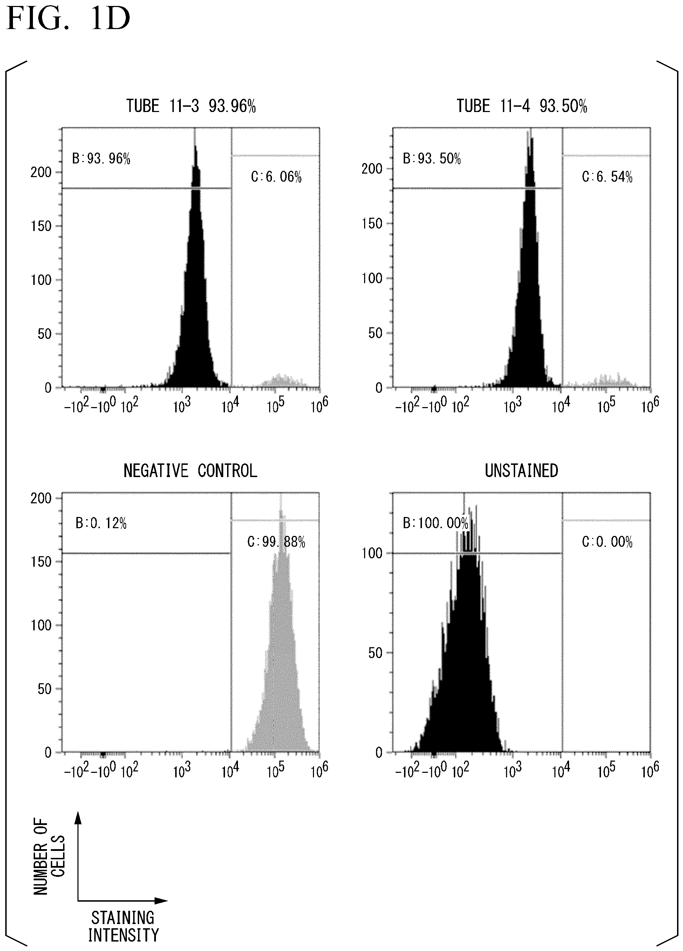

FIG. 1D is a graph showing the results of flow cytometric analysis in Examples.

### [Description of Embodiments]

### [Method for Producing Modified Human iPSCs]

In one embodiment, the present invention provides a method for producing modified human iPSCs in which two or more target genes have been modified, the production method including an introduction step of introducing CRISPR/Cas9 protein and a plurality of kinds of gRNAs for the two or more target genes into the human iPSCs.

In the production method of the present embodiment, the modification of the target genes may be introduction of a mutation into the target genes or may be disruption of the target genes, but is preferably the disruption of the target genes. In addition, the two or more target genes can be appropriately selected depending on an intended purpose, but are, for example, genes selected from the group consisting of HLA-A genes, HLA-B genes, HLA-C genes, HLA-DR genes, and Class II major histocompatibility complex transactivator (CIITA) genes.

The CIITA gene encodes a transcription factor that controls transcriptional activation of HLA Class II. Accordingly, cells in which the CIITA genes are disrupted do not express HLA class II protein, and have a reduced rejection reaction in a case where the cells are allogeneically transplanted into a recipient.

The NCBI accession number of a human CIITA gene is, for example, NM_000246.3, NM_001286402.1, NM_001286403.1.

In the production method of the present embodiment, the human iPSCs to be subjected to modification of genes are preferably blood cell-derived iPSCs. Since the blood cell-derived iPSCs can be obtained from cells in the blood that can be collected in a minimally invasive manner, they can be prepared relatively easily.

In the production method of the present embodiment, HLA-A genes or HLA-B genes in the human iPSCs to be subjected to modification of genes are preferably homozygous. In a case where both of the HLA-A genes and the HLA-B genes are heterozygous, in order to disrupt these genes, it is necessary to modify a total of four kinds of genes, that is two kinds of HLA-A genes and two kinds of HLA-B genes, for which a great deal of labor and creation time are required. On the other hand, in a case where the human iPSCs in which the HLA-A genes of the HLA-B genes are homozygous are used, labor and creation time taken for the modification of genes can be significantly reduced. In particular, it is preferable that both of the HLA-A genes and the HLA-B genes be homozygous since the labor and time required for the modification of genes can be reduced.

In the human iPSCs in which the HLA-A genes or the HLA-B genes are homozygous, it is preferable that haplotypes of the HLA-A genes include A24:02 and haplotypes of the HLA-B genes include B52:01.

In the human iPSCs, it is preferable that the HLA-A genes be homozygous and the haplotype of the HLA-A genes be A24:02. In addition, it is preferable that the HLA-B genes be homozygous and the haplotype of the HLA-B genes be B52:01.

In the human iPSCs, it is particularly preferable that both of the HLA-A genes and the HLA-B genes be homozygous, the haplotype of the HLA-A genes be A24:02, and the haplotype of the HLA-B genes be B52:01.

Moreover, in the human iPSCs, HLA-C genes are also preferably homozygous. In a case where the HLA-C genes remain, an attack by NK cells can be avoided. In addition, in a case where the HLA-C genes are homozygous, it is easier to match the HLA types to patients. In a case where the HLA-C genes are heterozygous, it is preferable to disrupt any one of the HLA-C genes.

The haplotypes in which the HLA-A genes, the HLA-B genes, and the HLA-C genes are all homozygous are shown in Table 1 below, in descending order of frequencies in Japanese people. The descending order of the frequencies of the HLA-C genes from the highest frequency is shown in Table 1.

**[Table 1]**

| Frequency (%) | Haplotype of HLA-C genes | Haplotypes of HLA-A genes and HLA-B genes |
|---|---|---|
| 17.28 | C01:02 | A24:02, B54:01 |
| 13.97 | C03:03 | A24:02, B35:01 |
| 12.71 | C07:02 | A24:02, B07:02 |
| 12.15 | C03:04 | A24:02, B40:02 |
| 10.87 | C12:02 | A24:02, B52:01 |
| 7.33 | C08:01 | A24:02, B40:06 |
| 6.8 | C14:02 | A31:01, B51:01 |
| 6.43 | C14:03 | A33:03, B44:03 |

From the viewpoint of facilitating the matching of HLA types with those of Japanese patients, human iPSCs in which HLA-A genes, HLA-B genes, and HLA-C genes are homozygous preferably have haplotypes of any of the following (i) to (iv), and particularly preferably have the haplotype of (i).
(i) The haplotype of the HLA-A genes is A24:02, the haplotype of the HLA-B genes is B52:01, and the haplotype of the HLA-C genes is C12:02.
(ii) The haplotype of the HLA-A genes is A24:02, the haplotype of the HLA-B genes is B54:01, and the haplotype of the HLA-C genes is C0L02.
(iii) The haplotype of the HLA-A genes is A24:02, the haplotype of the HLA-B genes is B07:02, and the haplotype of the HLA-C genes is C07:02.
(iv) The haplotype of the HLA-A genes is A33:03, the haplotype of the HLA-B genes is B44:03, and the haplotype of the HLA-C genes is C14:03.

In the production method of the present embodiment, HLA-DRB1 genes in the human iPSC to be subjected to modification of genes are preferably homozygous. In a case where the HLA-DRB1 genes are homozygous, it is easier to match the HLA types to patients.

In the production method of the present embodiment, the introduction step of introducing the CRISPR/Cas9 protein and the gRNAs into the human iPSCs is preferably performed by an electroporation method. As will be described later in Examples, in a case of using the electroporation method, modified human iPSCs in which two or more target genes have been modified can be produced with high efficiency.

Examples of the CRISPR/Cas9 protein include those derived from Pseudomonas aeruginosa, Staphylococcus aureus, Streptococcus thermophilus, and Geobacillus stearothermophilus. As the CRISPR/Cas9 protein, for example, a commercially available protein such as a product under a product name "Recombinant Cas9 Protein GMP grade" (Takara Bio Inc.) may be used.

Moreover, the gRNA is preferably a gRNA consisting of a base sequence set forth in SEQ ID NO: 1 and a gRNA consisting of a base sequence set forth in SEQ ID NO: 2. In this case, in the human iPSC to be subjected to modification of genes, the HLA-A genes and the HLA-B genes are homozygous, the haplotype of the HLA-A genes is A24:02, and the haplotype of the HLA-B genes is B52:01. Furthermore, instead of the gRNA consisting of the base sequence set forth in SEQ ID NO: 1, a gRNA consisting of a base sequence in which one or several bases are deleted, substituted, or added at a 5' end of the base sequence set forth in SEQ ID NO: 1 can also be used. Similarly, instead of the gRNA consisting of the base sequence set forth in SEQ ID NO: 2, a gRNA consisting of a base sequence in which one or several bases are deleted, substituted, or added at a 5' end of the base sequence set forth in SEQ ID NO: 2 can also be used. Here, the expression "one or several" may be, for example, 1 to 5, may be 1 to 4, may be 1 to 3, or may be 1 or 2.

The gRNA consisting of a base sequence set forth in SEQ ID NO: 1 is a shared single-guide RNA (sgRNA) for both the HLA-A genes and the HLA-B genes. The sgRNA consisting of the base sequence set forth in SEQ ID NO: 1 can modify both the HLA-A genes having a haplotype of A24:02 and the HLA-B genes having a haplotype of B52:01. In addition, the gRNA consisting of the base sequence set forth in SEQ ID NO: 2 is an sgRNA for the C11TA gene.

In the production method of the present embodiment, in the introduction step of introducing CRISPR/Cas9 protein and gRNAs into human iPSCs, the CRISPR/Cas9 protein, the gRNAs, and the human iPSCs may be mixed in any order.

The CRISPR/Cas9 protein and the human iPSCs may be mixed, and then the mixture is mixed with the gRNAs; the gRNAs and the human iPSCs are mixed, and then the mixture is mixed with the CRISPR/Cas9 protein; and the CRISPR/Cas9 protein and the gRNAs may be mixed, and then the mixture is mixed with the human iPSCs. Among these, it is preferable that the CRISPR/Cas9 protein and the gRNAs be mixed, and then the mixture be mixed with the human iPSCs. Accordingly, it is considered that a Cas9 protein-gRNA complex (RNP complex), which is a complex of the CRISPR/Cas9 protein and the gRNA, can be formed and introduced into the human iPSCs in the form of a complex. As will be described later in Examples, modified human iPSCs can be produced with high efficiency by introducing CRISPR/Cas9 protein and gRNAs in the form of a complex.

In the introduction step of introducing CRISPR/Cas9 protein and gRNAs into human iPSCs, it is preferable that the CRISPR/Cas9 protein and a plurality of kinds of gRNAs be introduced at a mass ratio of a mass of the CRISPR/Cas9 protein:a total mass of the plurality of kinds of gRNAs = 5:1 to 1:10.

The mass ratio of the mass of the CRISPR/Cas9 protein to the total mass of the plurality of kinds of gRNAs (the mass of the CRISPR/Cas9 protein:the total mass of the plurality of kinds of gRNAs) is preferably 3:1 to 1:10, more preferably 3:1 to 1:5, and particularly preferably 2:1 to 1:5. In addition, it is preferable to introduce the same mass of each of the plurality of kinds of gRNAs.

As will be described later in Examples, in a case where the mass ratio of the CRISPR/Cas9 protein to the gRNA is in the above-described range, modified human iPSCs can be produced with high efficiency.

Moreover, in the introduction step of introducing the CRISPR/Cas9 protein and the gRNAs into the human iPSCs, the amount of the CRISPR/Cas9 protein to be introduced is preferably 1 to 50 µg per 1 × 10⁵ to 3 × 10⁶ cells of the human iPSCs.

For example, the amount of the CRISPR/Cas9 protein to be introduced into 3 × 10⁵ cells of the human iPSCs may be 1 µg or more, may be 2 µg or more, may be 3 µg or more, or may be 4 µg or more. In addition, the amount of the CRISPR/Cas9 protein to be introduced into 3 × 10⁵ cells of the human iPSCs is preferably 10 µg or less, more preferably 9 µg or less, still more preferably 7 µg or less, and particularly preferably 5 µg or less. The upper limit value and the lower limit value can be randomly combined. Alternatively, the amount of the CRISPR/Cas9 protein to be introduced into 1.5 × 10⁶ cells of the human iPSCs may be 5 µg or more, may be 10 µg or more, may be 15 µg or more, or may be 20 µg or more. In addition, the amount of the CRISPR/Cas9 protein to be introduced into 1.5 × 10⁶ cells of the human iPSCs is preferably 50 µg or less, more preferably 40 µg or less, still more preferably 30 µg or less, and particularly preferably 25 µg or less. The upper limit value and the lower limit value can be randomly combined. As will be described later in Examples, in a case where the CRISPR/Cas9 protein is excessively present with respect to the gRNAs, a knockout efficiency tends to be decreased.

In the production method of the present embodiment, two or more target genes are genes selected from the group consisting of HLA-A genes, HLA-B genes, HLA-C genes, HLA-DR genes, and CIITA genes. According to the production method of the present embodiment, modified human iPSCs can be produced with high efficiency even in a case where any two or more target genes other than these genes are modified.

### [Modified Human iPSCs and Differentiated Cells Induced from Modified Human iPSCs]

In one embodiment, the present invention provides modified human iPSCs or differentiated cells induced from the modified human iPSCs, the cells having disrupted HLA-A genes, disrupted HLA-B genes, and disrupted CIITA genes, in which the disrupted HLA-A genes or the disrupted HLA-B genes are homozygous, haplotypes of the disrupted HLA-A genes include A24:02, and haplotypes of the disrupted HLA-B genes include B52:01.

In the human iPSCs or the differentiated cells, it is preferable that the HLA-A genes be homozygous and the haplotype of the HLA-A genes be A24:02. In addition, it is preferable that the HLA-B genes be homozygous and the haplotype of the HLA-B genes be B52:01.

In the human iPSCs or the differentiated cells, it is particularly preferable that both of the HLA-A genes and the HLA-B genes be homozygous, the haplotype of the HLA-A genes be A24:02, and the haplotype of the HLA-B genes be B52:01.

In another embodiment, the present invention provides modified human iPSCs or differentiated cells induced from the modified human iPSCs, the cells including disrupted HLA-A genes, disrupted HLA-B genes, and disrupted C11TA genes, in which haplotypes of HLA-C genes include C12:02.

In the modified human iPSCs and the differentiated cells induced from the iPSCs of the present embodiment, a rejection reaction is reduced in a case where the cells are allogeneically transplanted into a recipient. In addition, the rejection reaction of the differentiated cells induced from the modified human iPSCs of the present embodiment is also reduced in a case where the cells are allogeneically transplanted into a recipient. Accordingly, these cells can be suitably used for cell therapy and regenerative medicines in which pluripotent stem cells are differentiated into various cells and then transplanted into patients.

Moreover, in humans in which both the HLA-A genes and the HLA-B genes are homozygous, the haplotype of the HLA-A genes is A24:02, and the haplotype of the HLA-B genes is B52:01, the frequencies of expression in Japanese people are high. Therefore, in a case of producing the modified human iPSCs of the present embodiment, it is relatively easy to obtain human iPSCs to be subjected to modification of genes. In addition, as described above, in a case where the HLA-A genes or the HLA-B genes are homozygous, or both the HLA-A genes and HLA-B genes are homozygous, effort and production time taken for the modification of genes can be significantly reduced.

Moreover, as described above, the human iPSCs to be subjected to modification of genes are preferably blood cell-derived iPSCs. Since the blood cell-derived iPSCs can be obtained from cells in the blood that can be collected in a minimally invasive manner, they can be prepared relatively easily.

In the modified human iPSCs or the differentiated cells of the present embodiment, the expression "haplotypes of the disrupted HLA-A gene include A24:02 and haplotypes of the disrupted HLA-B gene include B52:01" means that although the HLA-A genes and the HLA-B genes are disrupted in a state for the HLA-A genes and the HLA-B genes unable to be expressed, the haplotypes of the HLA-A gene include A24:02 and the haplotypes of the HLA-B gene include B52:01.

In the modified human iPSCs or the differentiated cells of the present embodiment, it is preferable that the HLA-C genes remain. As described above, an attack by NK cells can be avoided in a case where the HLA-C genes remain. The haplotypes of HLA-C gene preferably include C12:02. In addition, the HLA-C genes are preferably homozygous. In a case where the HLA-C genes are homozygous, it is easier to match the HLA types to patients. In a case where the HLA-C genes are heterozygous, it is preferable to disrupt any one of the HLA-C genes.

### [Kit]

In one embodiment, the present invention provides a kit for modifying two or more target genes in human iPSCs by introducing into the human iPSCs, including CRISPR/Cas9 protein and a plurality of kinds of gRNAs for the two or more target genes. With the kit of the present embodiment, it is possible to suitably carry out the above-described method for producing modified human iPSCs.

In the kit of the present embodiment, the CRISPR/Cas9 protein is the same as described above.

In the kit of the present embodiment, the two or more target genes can be appropriately selected depending on the purpose thereof, but examples thereof include genes selected from the group consisting of HLA-A genes, HLA-B genes, HLA-C genes, HLA-DR genes, and CIITA genes. In this case, it is possible to produce human iPSCs in which a rejection reaction is reduced in a case where the iPSCs are allogeneically transplanted into a recipient.

In the kit of the present embodiment, the gRNA may be a gRNA consisting of a base sequence set forth in SEQ ID NO: 1 and a gRNA consisting of a base sequence set forth in SEQ ID NO: 2. In this case, in the human iPSC to be subjected to modification of genes, the HLA-A genes and the HLA-B genes are homozygous, the haplotype of the HLA-A genes is A24:02, and the haplotype of the HLA-B genes is B52:01. Furthermore, instead of the gRNA consisting of the base sequence set forth in SEQ ID NO: 1, a gRNA consisting of a base sequence in which one or several bases are deleted, substituted, or added at a 5' end of the base sequence set forth in SEQ ID NO: 1 can also be used. Similarly, instead of the gRNA consisting of the base sequence set forth in SEQ ID NO: 2, a gRNA consisting of a base sequence in which one or several bases are deleted, substituted, or added at a 5' end of the base sequence set forth in SEQ ID NO: 2 can also be used. Here, the expression "one or several" may be, for example, 1 to 5, may be 1 to 4, may be 1 to 3, or may be 1 or 2.

As described above, the gRNA consisting of the base sequence set forth in SEQ ID NO: 1 is a common sgRNA for both the HLA-A genes and the HLA-B genes. The sgRNA consisting of the base sequence set forth in SEQ ID NO: 1 can modify both the HLA-A genes having a haplotype of A24:02 and the HLA-B genes having a haplotype of B52:01. In addition, the gRNA consisting of the base sequence set forth in SEQ ID NO: 2 is an sgRNA for the CIITA gene.

In the kit of the present embodiment, it is preferable that the introduction of the CRISPR/Cas9 protein and the plurality of kinds of gRNAs into the human iPSCs be performed by an electroporation method.

Moreover, the kit of the present embodiment is preferably a kit used such that the CRISPR/Cas9 protein and the plurality of kinds of the gRNAs are introduced into the human iPSCs at a mass ratio of a mass of the CRISPR/Cas9 protein:a total mass of the plurality of kinds of gRNAs = 5:1 to 1:10.

The mass ratio of the mass of the CRISPR/Cas9 protein to the total mass of the plurality of kinds of gRNAs (the mass of the CRISPR/Cas9 protein:the total mass of the plurality of kinds of gRNAs) is preferably 3:1 to 1:10, more preferably 3:1 to 1:5, and particularly preferably 2:1 to 1:5. In addition, it is preferable to introduce the same mass of each of the plurality of kinds of gRNAs.

As will be described later in Examples, in a case where the mass ratio of the CRISPR/Cas9 protein to the gRNA is in the above-described range, modified human iPSCs can be produced with high efficiency.

Moreover, the kit of the present embodiment is preferably used to introduce the CRISPR/Cas9 protein in an amount of 1 to 50 µg per 1 × 10⁵ to 3 × 10⁶ cells of the human iPSCs.

For example, the amount of the CRISPR/Cas9 protein to be introduced into 3 × 10⁵ cells of the human iPSCs may be 1 µg or more, may be 2 µg or more, may be 3 µg or more, or may be 4 µg or more. In addition, the amount of the CRISPR/Cas9 protein to be introduced into 3 × 10⁵ cells of the human iPSCs is preferably 10 µg or less, more preferably 9 µg or less, still more preferably 7 µg or less, and particularly preferably 5 µg or less. The upper limit value and the lower limit value can be randomly combined. Alternatively, the amount of the CRISPR/Cas9 protein to be introduced into 1.5 × 10⁶ cells of the human iPSCs may be 5 µg or more, may be 10 µg or more, may be 15 µg or more, or may be 20 µg or more. In addition, the amount of the CRISPR/Cas9 protein to be introduced into 1.5 × 10⁶ cells of the human iPSCs is preferably 50 µg or less, more preferably 40 µg or less, still more preferably 30 µg or less, and particularly preferably 25 µg or less. The upper limit value and the lower limit value can be randomly combined. As will be described later in Examples, in a case where the CRISPR/Cas9 protein is excessively present with respect to the gRNAs, a knockout efficiency tends to be decreased.

In the kit of the present embodiment, the two or more target genes are genes selected from the group consisting of HLA-A genes, HLA-B genes, HLA-C genes, HLA-DR genes, and CIITA genes. According to kit of the present embodiment, modified human iPSCs can be produced with high efficiency even in a case where any two or more target genes other than these genes are modified.

### [Examples]

A more detailed description will be given of the present invention with reference to Examples, but the present invention is not limited to the following Examples.

Modified human iPSCs in which HLA-A genes, HLA-B genes, and CIITA genes of human iPSCs were deleted were produced by a CRISPR/Cas9 genome editing technique. Here, the conditions for the genome editing were examined.

As the human iPSCs, a Ff-I14s04 line at passage number 14 was used. In the Ff-I14s04 line, HLA-A genes, HLA-B genes, and HLA-C genes are homozygous. In addition, the haplotype of the HLA-A genes is A24:02, the haplotype of the HLA-B genes is B52:01, and the haplotype of the HLA-C genes is C12:02.

CRISPR/Cas9 protein and two kinds of gRNAs were introduced into the Ff-I14s04 line by an electroporation method. Electroporation was carried out using 4D-Nucleofector^{™} (Lonza K. K.).

As the gRNA, HLA-A24-ex2g1 (SEQ ID NO: 1), which is a common sgRNA for both the HLA-A genes and the HLA-B genes, and CIITA-ex3g5 (SEQ ID NO: 2), which is an sgRNA for the CIITA genes, were used.

Moreover, a 20 µL strip and a 100 µL cuvette were used as containers used for the electroporation. In addition, the mass ratio of the CRISPR/Cas9 protein to the gRNAs was variously changed.

First, the Ff-I14s04 line was released from the culture container. Subsequently, each of the cells was centrifuged at room temperature and 120 × g for 5 minutes, and the supernatant was removed.

Subsequently, in a case of using a 20 µL strip, the cells were suspended in 20 µL of a P3 Primary Cell Solution (Lonza K. K.) and a supplement (Lonza K. K.). In a case of using a 20 µL strip, 3 × 10⁵ cells per reaction were used.

Moreover, in a case of using a 100 µL cuvette, cells were suspended with 100 µL of a P3 Primary Cell Solution (Lonza K. K.) and a supplement (Lonza K. K.). In a case of using a 100 µL cuvette, 1.5 × 10⁶ cells were used per reaction.

Subsequently, with the combinations of Examples I to VI shown in Table 2 below, the CRISPR/Cas9 protein and the gRNAs were each mixed to form a Cas9 protein-gRNA complex (RNP complex), and then mixed with a cell suspension (n = 2). 20 µL strips were used for Examples I to V and a 100 µL cuvette was used for Example VI.

**[Table 2]**

| Example I | Concentration [ng/µL] | Tube 1 [µg] | Tube 2 [µg] |
|---|---|---|---|
| Genetically recombinant Cas9 protein | 3,000 | 5 | 5 |
| HLA-A24-ex2g1 | 1,656 | 1.25 | 1.25 |
| CIITA-ex3g5 | 1,460 | 1.25 | 1.25 |

| Example II | Concentration [ng/µL] | Tube 3 [µg] | Tube 4 [µg] |
|---|---|---|---|
| Genetically recombinant Cas9 protein | 3,000 | 5 | 5 |
| HLA-A24-ex2g1 | 1,656 | 5 | 5 |
| CIITA-ex3g5 | 1,460 | 5 | 5 |

| Example III | Concentration [ng/µL] | Tube 5 [µg] | Tube 6 [µg] |
|---|---|---|---|
| Genetically recombinant Cas9 protein | 3,000 | 5 | 5 |
| HLA-A24-ex2g1 | 1,656 | 12.5 | 12.5 |
| CIITA-ex3g5 | 1,460 | 12.5 | 12.5 |

| Example IV | Concentration [ng/µL] | Tube 7 [µg] | Tube 8 [µg] |
|---|---|---|---|
| Genetically recombinant Cas9 protein | 3,000 | 10 | 10 |
| HLA-A24-ex2g1 | 1,656 | 1.25 | 1.25 |
| CIITA-ex3g5 | 1,460 | 1.25 | 1.25 |

| Example V | Concentration [ng/µL] | Tube 9 [µg] | Tube 10 [µg] |
|---|---|---|---|
| Genetically recombinant Cas9 protein | 3,000 | 20 | 20 |
| HLA-A24-ex2g1 | 1,656 | 5 | 5 |
| CIITA-ex3g5 | 1,460 | 5 | 5 |

| Example VI | Concentration [ng/µL] | Tube 11 [µg] |
|---|---|---|
| Genetically recombinant Cas9 protein | 3,000 | 25 |
| HLA-A24-ex2g1 | 1,656 | 6.25 |
| CIITA-ex3g5 | 1,460 | 6.25 |

Subsequently, the 20 µL strip or the 100 µL cuvette was each set in a 4D-Nucleofector^{™} system (Lonza K. K.), a program CA-137 was selected, and electroporation was performed.

Subsequently, in a case of using the 20 µL strip, approximately 80 µL of a culture medium obtained by adding Y-27632 (CAS number: 129830-38-2) to AK03N (Ajinomoto Healthy Supply Co., Inc.) was taken from a well in the incubator and added into the cuvette, and the entire amount of cells inside the cuvette was seeded in one well of a 6-well plate.

Moreover, in a case of using the 100 µL cuvette, 20 µL was aspirated from the cuvette and seeded to one well of a 6-well plate (n = 4).

Subsequently, the day on which the cells after electroporation were seeded was defined as Day 0, and the initial culture medium exchange was performed on Day 2. Subsequently, in order to stabilize the cell state, one passage culture was performed. Passage was performed by adding one well for flow cytometric analysis. Subsequently, the cells were cryopreserved and the cells in a well for analysis were analyzed in the bulk state by flow cytometry.

Specifically, 50 ng/mL of IFN-γ was added to the culture medium of the iPSCs to perform a treatment for 48 hours, thereby inducing the expression of the HLA protein, and then stained with an anti-HLA-A2 antibody (product number: 740082, BD) to examine the expression of the HLA-A2 protein by flow cytometric analysis.

FIGS. 1A to 1D are graphs showing the results of flow cytometry analysis on each of the cells electroporated with Tubes 1 to 11 in Table 1. In FIGS. 1A to 1D, each of the vertical axes indicates the number of cells, and each of the horizontal axes indicates the staining intensity with an anti-HLA-A2 antibody. In addition, the "Negative control" shows the results obtained from the Ff-I14s04 line which was not genome-edited, and "Unstained" shows the representative results of cells not stained with the anti-HLA-A2 antibody.

Moreover, the ratios of the mass of the Cas9 protein to the total mass of the gRNAs, and the average values of the knockout efficiency of the HLA-A2 protein in each Example are shown in Table 3. Here, the negative rate of the HLA-A2 protein was defined as a knockout efficiency.

**[Table 3]**

| | Mass of Cas9 protein to total mass of gRNAs | Knockout efficiency (%) |
|---|---|---|
| Example I | 2:1 | 89.2 |
| Example II | 1:2 | 90.8 |
| Example III | 1:5 | 86.5 |
| Example IV | 4:1 | 53.6 |
| Example V | 2:1 | 77.6 |
| Example VI | 2:1 | 92.5 |

As a result, under the conditions other than Example IV, an extremely high knockout efficiency of about 80% or more could be obtained in any of the cases. The knockout efficiencies in Example IV (Tubes 7 and 8) were lower than those in other Examples, and a reason therefor was considered to be that the amount of the Cas9 protein was larger than that of the added gRNAs. Therefore, it was considered that there is a possibility that the knockout efficiency is decreased in a case where a large amount of a free Cas9 protein is present during formation of the RNP complex. Based on these results, it was considered that a balance between the mixing amounts of the gRNAs and the Cas9 protein is important for the knockout efficiency. In addition, from the comparison between the case of using the 20 µL strip and the case of using the 100 µL cuvette, it was revealed that the shape of the cuvette does not affect the knockout efficiency.

Moreover, as a result of comparison of the results of Tubes 11-1 to 11-4 of FIGS. 1C to 1D, it was found that in a case where a required amount (20 µL) was fractionated from the cuvette (100 µL) after the electroporation, a variation in knockout efficiency between cell populations at each fractionation run was not recognized.

From the results above, it was revealed that in order to increase or decrease the knockout efficiency, it is effective to adjust a balance between the mixing amounts of the gRNAs and the Cas9 protein. Furthermore, it was also revealed that the presence of excess Cas9 with respect to the optimized balance reduces the knockout efficiency. In addition, it was revealed that since the shape of the cuvette does not affect the knockout efficiency, any cuvette can be used as appropriate.

### [Industrial Applicability]

According to the present invention, it is possible to provide a technique for producing modified human iPSCs in which two or more target genes have been modified.

## Claims

1. A method for producing modified human induced pluripotent stem cells (iPSCs) in which two or more target genes have been modified, the production method comprising:
an introduction step of introducing CRISPR/Cas9 protein and a plurality of kinds of gRNAs for the two or more target genes into human iPSCs.

2. The production method according to Claim 1,
wherein the human iPSCs are blood cell-derived iPSCs.

3. The production method according to Claim 1 or 2,
wherein in the human iPSCs, HLA-A genes or HLA-B genes are homozygous.

4. The production method according to Claim 3,
wherein in the human iPSCs, haplotypes of the HLA-A genes include A24:02 and haplotypes of the HLA-B genes include B52:01.

5. The production method according to Claim 3 or 4,
wherein in the human iPSCs, HLA-C genes are homozygous.

6. The production method according to any one of Claims 3 to 5,
wherein in the human iPSCs, HLA-DRB1 genes are homozygous.

7. The production method according to any one of Claims 1 to 6,
wherein the introduction step is performed by an electroporation method.

8. The production method according to any one of Claims 1 to 7,
wherein the two or more target genes are genes selected from the group consisting of HLA-A genes, HLA-B genes, HLA-C genes, HLA-DR genes, and Class 11 major histocompatibility complex transactivator (CIITA) genes.

9. The production method according to any one of Claims 1 to 8,
wherein the gRNAs are
a gRNA consisting of a base sequence set forth in SEQ ID NO: 1 or a base sequence in which one or several bases are deleted, substituted, or added at a 5' end of the base sequence set forth in SEQ ID NO: 1, and
a gRNA consisting of a base sequence set forth in SEQ ID NO: 2 or a base sequence in which one or several bases are deleted, substituted, or added at a 5' end of the base sequence set forth in SEQ ID NO: 2.

10. The production method according to any one of Claims 1 to 9,
wherein in the introduction step, the CRISPR/Cas9 protein and the gRNAs are introduced in a form of a complex.

11. The production method according to any one of Claims 1 to 10,
wherein in the introduction step, the CRISPR/Cas9 protein and the plurality of kinds of the gRNAs are introduced at a mass ratio of a mass of the CRISPR/Cas9 protein:a total mass of the plurality of kinds of the gRNAs = 5:1 to 1:10.

12. The production method according to Claim 11,
wherein in the introduction step, the CRISPR/Cas9 protein is introduced in an amount of 1 to 50 µg per 1 × 10⁵ to 3 × 10⁶ cells of the human iPSCs.

13. The production method according to any one of Claims 1 to 12,
wherein the modification of the target genes is disruption of the target genes.

14. Modified human iPSCs or differentiated cells induced from the modified human iPSCs, the cells comprising:
disrupted HLA-A genes;
disrupted HLA-B genes; and
disrupted CIITA genes,
wherein the disrupted HLA-A genes or the disrupted HLA-B genes are homozygous,
haplotypes of the disrupted HLA-A genes include A24:02, and
haplotypes of the disrupted HLA-B genes include B52:01.

15. Modified human iPSCs or differentiated cells induced from the modified human iPSCs, the cells comprising:
disrupted HLA-A genes;
disrupted HLA-B genes; and
disrupted CIITA genes,
wherein haplotypes of HLA-C genes include C12:02.

16. A kit for modifying two or more target genes in human iPSCs by introducing into the human iPSCs, the kit comprising:
CRISPR/Cas9 protein; and
a plurality of kinds of gRNAs for the two or more target genes.

17. The kit according to Claim 16,
wherein the introduction is performed by an electroporation method.

18. The kit according to Claim 16 or 17,
wherein the two or more target genes are genes selected from the group consisting of HLA-A genes, HLA-B genes, HLA-C genes, HLA-DR genes, and CIITA genes.

19. The kit according to any one of Claims 16 to 18,
wherein the gRNAs are
a gRNA consisting of a base sequence set forth in SEQ ID NO: 1 or a base sequence in which one or several bases are deleted, substituted, or added at a 5' end of the base sequence set forth in SEQ ID NO: 1, and
a gRNA consisting of a base sequence set forth in SEQ ID NO: 2 or a base sequence in which one or several bases are deleted, substituted, or added at a 5' end of the base sequence set forth in SEQ ID NO: 2.

20. The kit according to any one of Claims 16 to 19,
wherein the kit is used to introduce the CRISPR/Cas9 protein and the plurality of kinds of the gRNAs into the human iPSCs at a mass ratio of a mass of the CRISPR/Cas9 protein:a total mass of the plurality of kinds of the gRNAs = 5:1 to 1:10.

21. The kit according to Claim 20,
wherein the kit is used to introduce the CRISPR/Cas9 protein in an amount of 1 to 50 µg per 1 × 10⁵ to 3 × 10⁶ cells of the human iPSCs.
